# EUROPÄISCHE PATENTSCHRIFT

(11) **EP 2 017 057 B1**
(45) Veröffentlichungstag und Bekanntmachung des Hinweises auf die Patenterteilung: **30.04.2014**
(21) Anmeldenummer: 07014113.0
(22) Anmeldetag: 18.07.2007
(51) Int. Cl.: B29C 44/12, A61F 11/08

(54) **Verfahren zum Herstellen eines einen Detektionskörper enthaltenden Ohrstöpsels aus Schaumkunststoff**
Method for manufacturing an ear plug made of foamed plastic and containing a detectable insert
Procédé destiné à la fabrication d'un embout auriculaire en mousse qui incorpore un objet détectable

(43) Veröffentlichungstag der Anmeldung: 21.01.2009
(73) Patentinhaber: Moldex-Metric AG & Co. KG, 72141 Walddorfhäslach (DE)
(72) Erfinder: Kern, Frank, 72124 Pilezhausen (DE); Armbruster, Peter, 72124 Pliezhausen (DE); Skov, Torben, 72070 Tübingen (DE)
(74) Vertreter: Grünecker, Kinkeldey, Stockmair & Schwanhäusser

(56) Entgegenhaltungen:
- EP-A- 0 786 241
- WO-A-00/40188
- FR-A1- 2 815 248
- US-A- 4 936 411
- US-A1- 2003 089 881

## Beschreibung

Die Erfindung bezieht sich auf ein Verfahren zum Herstellen eines Ohrstöpsels aus Schaumkunststoff nach dem Oberbegriff des Patentanspruchs 1.

Ohrstöpsel werden regelmäßig von Personen getragen, die mit der Produktion von gegen Fremdstoffe empfindlichen Erzeugnissen, beispielsweise Nahrungsmitteln oder Medikamenten, befasst sind. Dabei kann es vorkommen, dass die zum Gehörschutz getragenen Ohrstöpsel sich lösen und in das Erzeugnis gelangen. Um solche verlorenen Ohrstöpsel finden und vom Erzeugnis trennen zu können, ist es bekannt, die Ohrstöpsel mit einem berührungsfrei detektierbaren Detektionskörper zu versehen. Dabei kann es sich beispielsweise um mittels Magnet- oder Metallsensoren oder mittels Ultraschall oder Röntgenstrahlen berührungsfrei feststellbare Körper handeln.

Bei einem aus der EP 1 061 875 B1 oder der WO 90/01914 bekannten Verfahren dieser Art wird zunächst der Ohrstöpsel geschäumt. Anschließend wird mittels eines Stanzwerkzeugs oder einer Nadel vom Rückseitenende her in den Ohrstöpsel ein Kanal eingeformt, in den schließlich der Detektionskörper eingebracht wird. Damit sich der Kanal nicht zu schnell durch die Rückverformung des Schaumstoffes zusetzt, schlägt die EP 1 061 875 B1 vor, das kanalbildende Werkzeug oder den Ohrstöpselkörper zu kühlen. Die Kanalbildung stellt einen zusätzlichen Arbeitsgang dar.

Aus der WO 2005/104980 A2 mit der FR 2 815 248 A1 ist es bekannt, beim Herstellen von Ohrstöpseln aus Schaumstoff in die Schaummasse sehr kleine detektierbare Partikel einzubringen, die sich beim Aufschäumen im Schaumkunststoff verteilen. Hier hat man jedoch keine Kontrolle über die Lage der detektierbaren Partikel im Ohrstöpsel. Es kann daher vorkommen, dass sich die Partikel aufgrund ihres Gewichts im Einsteckende des Ohrstöpsels sammeln und dadurch das Einführen des Ohrstöpsels in den Gehörgang erschweren. Die detektierbaren Partikel können auch an beliebigen Stellen der Oberfläche des Ohrstöpsels zum Liegen kommen, wodurch sie mit der Haut des Benutzers in Berührung kommen und zu Irritationen führen können. Daher schlägt die WO 2005/104980 A2 vor, den Ohrstöpsel in zwei Schritten dadurch zu erzeugen, dass zunächst eine von detektierbaren Partikeln freie Außenhaut gebildet und in dieser der mit detektierbaren Partikeln durchsetzte Hauptkörper des Ohrstöpsels aufgeschäumt wird. Dies stellt einen zusätzlichen Arbeitsgang dar. Außerdem sind sehr kleinteilige, pulverförmige Detektionspartikel schwerer zu detektieren als ein zusammenhängender, größerer Detektionskörper.

Aus der WO 00/40188 A ist es bekannt, einen Detektionskörper in den Schaumstoff entweder dadurch einzubringen, dass mittels eines Stösels ein Hohlraum in den Schaumstoffkörper eingestanzt und der Detektionskörper in diesen Hohlraum eingesetzt wird oder dadurch, dass der Detektionskörper mit kinetischer Energie in den Schaumstoff hineingeschossen wird. Im ersteren Fall bildet der gestanzte Hohlraum und im letzteren Fall der Einschusskanal des Detektionskörpers im Schaumstoff eine Inhomogenität, sodass der im Hohlraum oder im Schusskanal befindliche Detektionskörper nicht homogen vom Schaumstoff umgeben ist.

Der Erfindung liegt die Aufgabe zugrunde, ein Verfahren der eingangs definierten Gattung zu schaffen, bei dem auf einfache Weise ein leicht detektierbarer Detektionskörper an gewünschter Stelle im Ohrstöpsel platziert werden kann.

Diese Aufgabe wird bei einem gattungsgemäßen Verfahren durch die kennzeichnenden Merkmale des Patentanspruchs 1 gelöst.

Durch die Erwärmung des Detektionskörpers auf eine Temperatur, die oberhalb der beim Aufschäumen im Schaumkunststoff herrschenden Temperatur liegt, wird der den Detektionskörper beim Einsinken in den aufschäumenden Kunststoff unmittelbar umgebende Kunststoff durch Wärmeabgabe vom Detektionskörper auf ein höheres Temperaturniveau gebracht, wodurch das Aufschäumen und die im Schaumstoff stattfindende Polymerisation beschleunigt werden. Dadurch bildet sich in dem im Aufschäumen begriffenen Kunststoff eine bereits weitgehend oder ganz ausgeschäumte, im Vergleich zum flüssigen Schaumstoff feste Porenstruktur, die sich dem weiteren Einsinken des Detektionskörpers in die Schaummasse widersetzt. Dadurch bleibt der Detektionskörper im Schaumkunststoff in Schwebe und hält einen Abstand vom Einsteckende des Ohrstöpsels. Eines nachträglichen Einbringens eines Einsteckkanals bedarf es hierzu nicht.

Als Schaumkunststoff wird zweckmäßigerweise ein üblicher Zweikomponenten-PolyurethanKunststoff verwendet, der aufschäumt, sobald die beiden Komponenten in Berührung miteinander kommen. Dabei wird, wie bei Schaumkunststoff-Ohrstöpseln üblich, bevorzugt ein Schaumkunststoff verwendet, der nach einer elastischen Verformung nur allmählich und mit zeitlicher Verzögerung seine ursprüngliche Form wieder einnimmt.

Die Einsinktiefe des Detektionskörpers in den Ohrstöpsel und damit der Abstand, den der Detektionskörper vom Einsteckende des Ohrstöpsels hält, lässt sich durch Wahl der Temperatur des Detektionskörpers und durch Bestimmung des Zeitpunkts während des Aufschäumvorgangs, an dem der Detektionskörper in den Schaum eingebracht wird, kontrollieren. Die konkrete Temperatur und der konkrete Einbringzeitpunkt lassen sich empirisch feststellen.

Als zweckmäßig hat sich erwiesen, wenn der Detektionskörper auf ca. 40 - 80° C, vorzugsweise ca. 50 - 70° C, und äußerst bevorzugt auf ca. 60° C oberhalb der beim Aufschäumen herrschenden Temperatur erhitzt wird.

Wenn beim Aufschäumen eine Temperatur von ca. 40° C herrscht, ist der Detektionskörper also auf ca. 80 -120° C, vorzugsweise 90 -110° C und äußerst bevorzugt auf ca. 100° C zu erwärmen.

Die Temperatur des Detektionskörpers und der Zeitpunkt des Einbringens werden zweckmäßigerweise so gewählt, dass der Detektionskörper mit seinem Schwerpunkt näher beim Rückseiten- als beim Einsteckende des Ohrstöpsels zum Stillstand kommt. Hierdurch ist gewährleistet, dass der Detektionskörper das Einführen des Ohrstöpsels in den Gehörgang nicht erschwert.

Dabei ist es besonders günstig, wenn der Detektionskörper zu einem solchen Zeitpunkt des Aufschäumens und mit einer solchen Temperatur in den Schaumkunststoff eingebracht wird, dass er in seiner Stillstandslage allseitig vom Schaumkunststoff eingehüllt ist. Es schadet zwar nicht, wenn der Detektionskörper am Rückseitenende des Ohrstöpsels oder an den seitlichen Begrenzungsflächen im Abstand vom Einsteckende etwas hervorsteht; um einen Verlust des Detektionskörpers zuverlässig zu verhindern und um einen Kontakt zwischen der Haut des Benutzers und dem Detektionskörper zu vermeiden, ist es jedoch besser, wenn der Detektionskörper allseitig vom Schaumkunststoff umgeben ist.

Vorzugsweise wird der Detektionskörper zu einem solchen Zeitpunkt des Aufschäumens mit einer solchen Temperatur in den Schaumkunststoff eingebracht, dass der Detektionskörper in seiner Stillstandslage zum Rückseitenende einen Abstand von ca. 1 - 5 mm hat. Der Detektionskörper ist dann zum Rückseitenende hin ausreichend abgedeckt, um nicht verloren gehen zu können, hat aber andererseits einen so großen Abstand zum Einsteckende, dass er sich beim Einführen des Ohrstöpsels in den Gehörgang nicht bemerkbar macht.

Nach dem Einbringen des Detektionskörpers wird die Form vorteilhafterweise geschlossen. Dadurch bildet sich eine weitgehend ebene Fläche am Rückseitenende des Ohrstöpsels aus.

Das Volumen des Detektionskörpers ist so zu wählen, dass er einerseits einwandfrei detektierbar ist und andererseits die Handhabung des Ohrstöpsels, insbesondere das Einführen in den Gehörgang, nicht behindert. Als zweckmäßig hat sich ein Volumen von ca. 0,2 - ca. 15 mm³, vorzugsweise ca. 3 - ca. 5 mm³ und äußerst bevorzugt von ca. 4 mm³ gezeigt.

Der Detektionskörper hat vorzugsweise die Form einer Kugel, wobei sich Durchmesser von ca. 0,7 - ca. 3 mm und äußerst bevorzugt von ca. 2 mm bewährt haben.

Als Material für den Detektionskörper eignet sich Stahl und insbesondere nicht-rostender Stahl.

Besonders gut lässt sich die Einsinktiefe des Detektionskörpers in den Schaumkunststoff kontrollieren, wenn dessen Oberfläche aufgeraut ist, was beispielsweise dadurch erreicht werden kann, dass der Detektionskörper mit Säure behandelt wird.

Mit dem erfindungsgemäßen Verfahren wird erstmalig ein Ohrstöpsel aus Schaumkunststoff geschaffen, der einen Detektionskörper eines Volumens von größer als 0,2 - ca. 15 mm³ enthält, der homogen von Schaumkunststoff umgeben ist. Bei den bekannten Ohrstöpseln mit einem Detektionskörper dieses Volumens ist die Homogenität des Schaumkunststoffes benachbart zur Oberfläche des Detektionskörpers unterbrochen durch den in den Schaumkunststoff eingearbeiteten Einführungskanal für den Detektionskörper.

Das erfindungsgemäße Verfahren und der damit erzeugte Ohrstöpsel werden nachstehend anhand einer schematischen Schnittdarstellung einer zum Aufschäumen verwendeten Form erläutert.

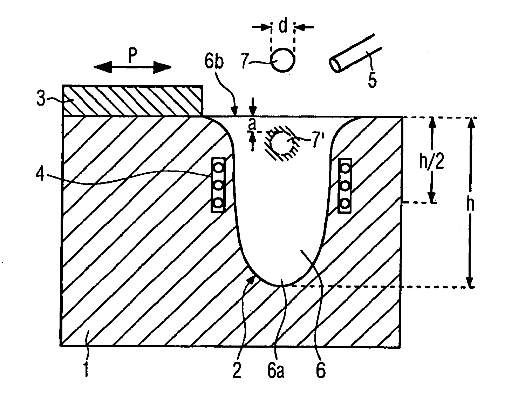

In der Zeichnung ist 1 eine Schäumform mit einem der Kontur des zu erzeugenden Ohrstöpsels entsprechenden Schäumhohlraum 2. Der längliche Schäumhohlraum 2 ist im Wesentlichen vertikal ausgerichtet und nach oben offen. Er kann jedoch durch einen Abschlussdeckel 3, der im Sinne des Doppelpfeils P hin- und herbewegbar ist, nach oben zu abgeschlossen werden.

Der Schäumhohlraum 2 ist von einer Heizeinrichtung 4 umgeben, mit der die Oberfläche des Schäumhohlraums 2 und der darin befindliche Schaumstoff erwärmt werden können.

Durch eine Zuführleitung 5 wird in den Schäumhohlraum 2 ein Zweikomponenten-Polyurethan-Kunststoff eingefüllt. Durch den Kontakt der beiden Komponenten schäumt der Kunststoff in bekannter Weise sofort auf. Der verwendete Polyurethan-Schaumstoff ist von dem Typ, der sich nach einer Verformung nur langsam zurückbildet, wie er häufig für bekannte Ohrstöpsel eingesetzt wird.

Denkbar ist aber auch der Einsatz anderer Schaumstoffe.

Durch das Aufschäumen bildet sich im Inneren des Schäumhohlraums 2 ein Schaumstoff-Ohrstöpsel 6, der ein sich verjüngendes Einsteckende 6a und ein im Wesentliches ebenes Rückseitenende 6b besitzt.

In den aufschäumenden Kunststoff wird von oben ein Detektionskörper 7 so eingebracht, dass er frei in die aufschäumende Kunststoffmasse einsinken kann. Dieser Detektionskörper, der im gezeigten Ausführungsbeispiel eine Kugel vom Durchmesser d ist, kann aus jedem berührungsfrei detektierbaren Material bestehen. Insbesondere kommen Metalle in Frage, die mittels eines Metalldetektors gefunden werden können. Verwendbar sind auch insbesondere magnetisierbare Metalle oder andere magnetisierbare Stoffe, die mittels Magnetsensoren detektierbar sind. Es kommen aber auch andere Stoffe, wie beispielsweise Keramik, und andere Detektionsmittel, wie beispielsweise Ultraschall oder Röntgenstrahlen, in Frage.

Bei dem bevorzugten Ausführungsbeispiel besteht der kugelförmige Detektionskörper 7 aus nicht-rostendem Stahl, dessen Oberfläche in einem Salzsäurebad aufgeraut wurde, wobei die Tiefe der durch die Säure gebildeten Löcher ca. 5 - 50 µm beträgt.

Der Detektionskörper 7 wird vor dem Einbringen in den Schäumhohlraum 2 auf eine Temperatur erhitzt, die oberhalb der im aufschäumenden Kunststoff herrschenden Temperatur liegt.

Das Einführen des Detektionskörpers 7 in den Schäumhohlraum 2 findet nach Beginn des Aufschäumvorgangs und zweckmäßigerweise kurz vor Beendigung des Aufschäumens, beispielsweise wenn der Schaum ca. 80 % des Volumens des Schäumhohlraums 2 ausgefüllt hat, statt.

Die Temperaturdifferenz zwischen dem erhitzten Detektionskörper 7 und dem aufschäumenden Kunststoff sowie der Zeitpunkt des Einbringens des Detektionskörpers 7 in den Kunststoff werden so gewählt, dass der Schaumstoff durch Wärmeübertragung vom Detektionskörper 7 aus in der Umgebung des in den Schaumstoff einsinkenden Detektionskörpers schneller ausschäumt und aushärtet (auspolymerisiert) als im übrigen Schäumhohlraumbereich. Die sich dabei ausbildende Porenstruktur behindert das weitere Einsinken des Detektionskörpers in den Schaumstoff und bringt den Einsinkvorgang schließlich zum Stillstand. Bei dem hier beschriebenen Ausführungsbeispiel wird der Detektionskörper 7 auf ca. 60° C oberhalb der im aufschäumenden Kunststoff herrschenden Temperatur erwärmt und in den aufschäumenden Kunststoff eingeführt, sobald dieser fast die obere Begrenzungsfläche des Schäumhohlraums 2 erreicht hat.

Idealerweise werden die Temperatur und der Einbringzeitraum so gewählt, dass der Detektionskörper 7 etwa in der gestrichelt in der Zeichnung dargestellten Lage 7' zu liegen kommt. Diese Lage befindet sich oberhalb der halben Höhe h des Schäumhohlraums 2, welche Höhe h üblicherweise und im beschriebenen Ausführungsbeispiel 22 - 23 mm beträgt, und unterhalb der Rückseitenendfläche 6b. Der Abstand a zur Rückseitenoberfläche 6b sollte ca. 1 - 5 mm betragen, kann aber auch größer sein, solange zum Einsteckende 6a des Ohrstöpsels 6 ein ausreichend großer Abstand besteht, um sicherzustellen, dass der Detektionskörper 7 beim Einführen des Ohrstöpsels in den Gehörgang nicht stört.

Der Temperaturunterschied zwischen dem erhitzten Detektionskörper 7 und dem aufschäumenden Schaumstoff kann auch höher oder niedriger als vorstehend angegeben liegen, sollte aber den Bereich von 40 - 80° C nicht unter- bzw. überschreiten. Der tatsächliche Temperaturbereich für den Detektionskörper liegt zwischen ca. 80 - ca. 120° C.

Für den Detektionskörper kommen auch andere Raumformen als die Kugel in Frage. Dabei sollte aber ein Volumen von ca. 0,2 - ca. 15 mm³ nicht unter- bzw. überschritten werden. Bevorzugt ist ein Bereich von ca. 3 - ca. 5 mm³ und optimal ein Volumen von ca. 4 mm³.

Im beschriebenen Ausführungsbeispiel beträgt der Kugeldurchmesser d ca. 2 mm, kann aber von ca. 0,7 - ca. 3 mm variieren.

Bei dem nach dem erfindungsgemäßen Verfahren hergestellten Ohrstöpsel liegt der Detektionskörper 7 in der Lage 7' in einer ihn umgebenden in der Zeichnung gestrichelt angedeuteten Schaumstoffschicht, die homogen ist, also keine durch Einschnitte oder Ausstanzungen gebildeten Inhomogenitäten aufweist. Idealerweise ist der Detektionskörper an der Stellung 7' ringsum von Schaumstoff umhüllt. Die Verwendbarkeit des Ohrstöpsels wird aber nicht entscheidend beeinträchtigt, wenn der Detektionskörper nach oben über die Rückseitenendfläche 6b oder über die Seitenflächen etwas hinausragt. Sichergestellt muss nur sein, dass er tief genug im Schaumstoff verankert ist, um nicht verloren gehen zu können und dass vom Einsteckende 6a ein ausreichender Abstand gehalten ist, um die Einführbarkeit des Ohrstöpsels in den Gehörgang nicht zu beeinträchtigen.

## Patentansprüche

1. Verfahren zum Herstellen eines ein Einsteck- und ein Rückseitenende (6a bzw. 6b) aufweisenden sowie einen berührungslos detektierbaren Detektionskörper (7) im Inneren im Abstand vom Einsteckende (6a) enthaltenden Ohrstöpsels (6), bei dem Schaumkunststoff in einer das Einsteckende (6a) unten und das Rückseitenende (6b) oben bildenden Form (1) zum Aufschäumen gebracht und der Detektionskörper (7) in den Schaumkunststoff eingebracht wird, **dadurch gekennzeichnet, dass** der Detektionskörper (7) während des Aufschäumens von oben in die offene Form (1) zum freien Einsinken in den Schaumkunststoff eingebracht und vor dem Einbringen auf eine Temperatur erhitzt wird, die so weit oberhalb der beim Aufschäumen im Schaumkunststoff herrschenden Temperatur liegt, dass die Einsinkbewegung des Detektionskörpers (7) im Abstand oberhalb des Einsteckendes zum Stillstand kommt.

2. Verfahren nach Anspruch 1, **dadurch gekennzeichnet, dass** der Schaumkunststoff ein Zweikomponenten-Polyurethankunststoff ist.

3. Verfahren nach Anspruch 1 und/oder 2, **dadurch gekennzeichnet, dass** der Detektionskörper (7) auf ca. 40 - 80° C oberhalb der beim Aufschäumen herrschenden Temperatur erhitzt wird.

4. Verfahren nach Anspruch 3, **dadurch gekennzeichnet, dass** der Detektionskörper (7) auf ca. 50 - 70° C oberhalb der beim Aufschäumen herrschenden Temperatur erhitzt wird.

5. Verfahren nach Anspruch 4, **dadurch gekennzeichnet, dass** der Detektionskörper (7) auf ca. 60° C oberhalb der beim Aufschäumen herrschenden Temperatur erhitzt wird.

6. Verfahren nach einem der Ansprüche 1 bis 5, **dadurch gekennzeichnet, dass** der Detektionskörper (7) auf ca. 80 - 120° C erhitzt wird.

7. Verfahren nach Anspruch 6, **dadurch gekennzeichnet, dass** der Detektionskörper (7) auf 90 -110° C erhitzt wird.

8. Verfahren nach Anspruch 7, **dadurch gekennzeichnet, dass** der Detektionskörper (7) auf ca. 100° C erhitzt wird.

9. Verfahren nach einem der Ansprüche 1 bis 8, **dadurch gekennzeichnet, dass** der Detektionskörper (7) zu einem solchen Zeitpunkt des Aufschäumens und mit einer solchen Temperatur in den Schaumkunststoff eingebracht wird, dass der Detektionskörper (7) mit seinem Schwerpunkt näher beim Rückseiten- als beim Einsteckende zum Stillstand kommt.

10. Verfahren nach einem der Ansprüche 1 bis 9, **dadurch gekennzeichnet, dass** der Detektionskörper (7) zu einem solchen Zeitpunkt des Aufschäumens und mit einer solchen Temperatur in den Schaumkunststoff eingebracht wird, dass der Detektionskörper (7) in seiner Stillstandslage (7') allseitig vom Schaumkunststoff eingehüllt ist.

11. Verfahren nach einem der Ansprüche 1 bis 10, **dadurch gekennzeichnet, dass** der Detektionskörper (7) zu einem solchen Zeitpunkt des Aufschäumens und mit einer solchen Temperatur in den Schaumkunststoff eingebracht wird, dass der Detektionskörper (7) in seiner Stillstandslage (7') zum Rückseitenende (6b) einen Abstand (a) von ca. 1 bis 5 mm hat.

12. Verfahren nach einem der Ansprüche 1 bis 11, **dadurch gekennzeichnet, dass** die Form (1) nach dem Einbringen des Detektionskörpers (7) geschlossen wird.

13. Verfahren nach einem der Ansprüche 1 bis 12, **dadurch gekennzeichnet, dass** der Detektionskörper (7) ein Volumen von ca. 0,2 bis ca. 15 mm³ hat.

14. Verfahren nach Anspruch 13, **dadurch gekennzeichnet, dass** der Detektionskörper (7) ein Volumen von ca. 3 - ca. 5 mm³ hat.

15. Verfahren nach Anspruch 14, **dadurch gekennzeichnet, dass** der Detektionskörper (7) ein Volumen von ca. 4 mm³ hat.

16. Verfahren nach einem der Ansprüche 1 bis 15, **dadurch gekennzeichnet, dass** der Detektionskörper (7) eine Kugel ist.

17. Verfahren nach Anspruch 16, **dadurch gekennzeichnet, dass** die Kugel (7) einen Durchmesser (d) von ca. 0,7 - ca. 3 mm hat.

18. Verfahren nach Anspruch 17, **dadurch gekennzeichnet, dass** die Kugel (7) einen Durchmesser (d) von ca. 2 mm hat.

19. Verfahren nach einem der Ansprüche 1 bis 18, **dadurch gekennzeichnet, dass** der Detektionskörper (7) aus Stahl, vorzugsweise nicht-rostendem Stahl, besteht.

20. Verfahren nach einem der Ansprüche 1 bis 19, **dadurch gekennzeichnet, dass** die Oberfläche des Detektionskörpers (7) aufgeraut ist.

21. Verfahren nach Anspruch 20, **dadurch gekennzeichnet, dass** die Oberfläche durch Einlegen des Detektionskörpers (7) in Säure aufgeraut wird.

22. Verfahren nach Anspruch 20, **dadurch gekennzeichnet, dass** die Rautiefe der Oberfläche ca. 5 - 50 µm beträgt.

## Claims

1. Method of producing an ear plug (6) having a plug-in end and a rear face end (6a respectively 6b) and containing a contactlessly detectable detection body (7) in the interior at a distance from the plug-in end (6a), whereby for the foaming process, cellular plastic is placed in a mould (1) forming the plug-in end (6a) at the bottom and the rear face end (6b) at the top and the detection body (7) is introduced into the cellular plastic, **characterised in that** the detection body (7) is introduced into the open mould (1) from the top during the foaming process so that it sinks freely into the cellular plastic and prior to being introduced is heated to a temperature which is so far above the temperature prevailing in the cellular plastic during the foaming process that the sinking movement of the detection body (7) comes to a halt at a distance above the plug-in end.

2. Method as claimed in claim 1, **characterised in that** the cellular plastic is a two-component polyurethane plastic.

3. Method as claimed in claim 1 and/or 2, **characterised in that** the detection body (7) is heated to approximately 40 - 80° C above the temperature prevailing during the foaming process.

4. Method as claimed in claim 3, **characterised in that** the detection body (7) is heated to approximately 50 - 70° C above the temperature prevailing during the foaming process.

5. Method as claimed in claim 4, **characterised in that** the detection body (7) is heated to approximately 60° C above the temperature prevailing during the foaming process.

6. Method as claimed in one of claims 1 to 5, **characterised in that** the detection body (7) is heated to approximately 80 - 120° C.

7. Method as claimed in claim 6, **characterised in that** the detection body (7) is heated to 90 - 110° C.

8. Method as claimed in claim 7, **characterised in that** the detection body (7) is heated to approximately 100° C.

9. Method as claimed in one of claims 1 to 8, **characterised in that** the detection body (7) is introduced into the cellular plastic at an instant of the foaming process and at a temperature such that the detection body (7) comes to a halt with its centre of gravity closer to the rear face end than to the plug-in end.

10. Method as claimed in one of claims 1 to 9, **characterised in that** the detection body (7) is introduced into the cellular plastic at an instant of the foaming process and at a temperature such that the detection body (7) is encased in the cellular plastic on all sides in its halted position (7').

11. Method as claimed in one of claims 1 to 10, **characterised in that** the detection body (7) is introduced into the cellular plastic at an instant of the foaming process and at a temperature such that the detection body (7) is at a distance (a) of approximately 1 to 5 mm from the rear face end (6b) in its halted position (7').

12. Method as claimed in one of claims 1 to 11, **characterised in that** the mould (1) is closed after the detection body (7) has been introduced.

13. Method as claimed in one of claims 1 to 12, **characterised in that** the detection body (7) has a volume of approximately 0.2 to approximately 15 mm³.

14. Method as claimed in claim 13, **characterised in that** the detection body (7) has a volume of approximately 3 to approximately 5 mm³.

15. Method as claimed in claim 14, **characterised in that** the detection body (7) has a volume of approximately 4 mm³.

16. Method as claimed in one of claims 1 to 15, **characterised in that** the detection body (7) is a sphere.

17. Method as claimed in claim 16, **characterised in that** the sphere (7) has a diameter (d) of approximately 0.7 to approximately 3 mm.

18. Method as claimed in claim 17, **characterised in that** the sphere (7) has a diameter (d) of approximately 2 mm.

19. Method as claimed in one of claims 1 to 18, **characterised in that** the detection body (7) is made from steel, preferably stainless steel.

20. Method as claimed in one of claims 1 to 19, **characterised in that** the surface of the detection body (7) is roughened.

21. Method as claimed in claim 20, **characterised in that** the surface is roughened by immersing the detection body (7) in acid.

22. Method as claimed in claim 20, **characterised in that** the roughness depth of the surface is approximately 5 - 50 µm.

## Revendications

1. Procédé de fabrication d'un bouchon d'oreille (6) ou embout auriculaire présentant une extrémité d'introduction et une extrémité de côté arrière (6a respectivement 6b) et renfermant intérieurement un corps détectable (7), qui peut être détecté sans contact et est situé à distance de l'extrémité d'introduction (6a), procédé
d'après lequel de la matière plastique pour mousse est amenée dans un moule (1) formant l'extrémité d'introduction (6a) dans le bas et l'extrémité de côté arrière (6b) dans le haut, en vue d'y être moussée, et le corps détectable (7) est introduit dans la matière plastique sous forme de mousse,
**caractérisé en ce que** le corps détectable (7) est introduit par le haut dans le moule (1) ouvert, pour s'enfoncer librement dans la matière plastique en mousse, et est chauffé, avant son introduction, à une température, qui se situe au-dessus de la température régnant dans la matière plastique en mousse lors du moussage, de manière telle que le mouvement d'enfoncement libre du corps détectable (7) s'interrompe en conduisant à l'immobilisation du corps détectable à distance au-dessus de l'extrémité d'introduction.

2. Procédé selon la revendication 1, **caractérisé en ce que** la matière plastique sous forme de mousse est une matière plastique de polyuréthanne à deux composants.

3. Procédé selon la revendication 1 et/ou la revendication 2, **caractérisé en ce que** le corps détectable (7) est échauffé à une température d'environ 40 - 80°C au-dessus de la température régnant lors du moussage.

4. Procédé selon la revendication 3, **caractérisé en ce que** le corps détectable (7) est échauffé à une température d'environ 50 - 70°C au-dessus de la température régnant lors du moussage.

5. Procédé selon la revendication 4, **caractérisé en ce que** le corps détectable (7) est échauffé à une température d'environ 60°C au-dessus de la température régnant lors du moussage.

6. Procédé selon l'une des revendications 1 à 5, **caractérisé en ce que** le corps détectable (7) est échauffé à une température d'environ 80 - 120°C.

7. Procédé selon la revendication 6, **caractérisé en ce que** le corps détectable (7) est échauffé à une température d'environ 90 - 110°C.

8. Procédé selon la revendication 7, **caractérisé en ce que** le corps détectable (7) est échauffé à une température d'environ 100°C.

9. Procédé selon l'une des revendications 1 à 8, **caractérisé en ce que** le corps détectable (7) est introduit dans la matière plastique sous forme de mousse, à un instant tel du moussage et avec une température telle, que le corps détectable (7) vienne à s'immobiliser avec son centre de gravité plus proche de l'extrémité de côté arrière que de l'extrémité d'introduction.

10. Procédé selon l'une des revendications 1 à 9, **caractérisé en ce que** le corps détectable (7) est introduit dans la matière plastique sous forme de mousse, à un instant tel du moussage et avec une température telle, que le corps détectable (7), dans sa position d'immobilisation (7'), soit enveloppé de toutes parts par la matière plastique sous forme de mousse.

11. Procédé selon l'une des revendications 1 à 10, **caractérisé en ce que** le corps détectable (7) est introduit dans la matière plastique sous forme de mousse, à un instant tel du moussage et avec une température telle, que le corps détectable (7), dans sa position d'immobilisation (7'), présente une distance d'espacement (a) de l'extrémité de côté arrière (6b) d'environ 1 à 5 mm.

12. Procédé selon l'une des revendications 1 à 11, **caractérisé en ce que** le moule (1) est fermé après l'introduction du corps détectable (7).

13. Procédé selon l'une des revendications 1 à 12, **caractérisé en ce que** le corps détectable (7) présente un volume d'environ 0,2 à environ 15 mm³.

14. Procédé selon la revendication 13, **caractérisé en ce que** le corps détectable (7) présente un volume d'environ 3 - environ 5 mm³.

15. Procédé selon la revendication 14, **caractérisé en ce que** le corps détectable (7) présente un volume d'environ 4 mm³.

16. Procédé selon l'une des revendications 1 à 15, **caractérisé en ce que** le corps détectable (7) est une bille.

17. Procédé selon la revendication 16, **caractérisé en ce que** la bille (7) présente un diamètre (d) d'environ 0,7 - environ 3 mm.

18. Procédé selon la revendication 17, **caractérisé en ce que** la bille (7) présente un diamètre (d) d'environ 2 mm.

19. Procédé selon l'une des revendications 1 à 18, **caractérisé en ce que** le corps détectable (7) est réalisé en acier, de préférence en acier inoxydable.

20. Procédé selon l'une des revendications 1 à 19, **caractérisé en ce que** la surface extérieure du corps détectable (7) est rendue rugueuse.

21. Procédé selon la revendication 20, **caractérisé en ce que** la surface extérieure est rendue rugueuse en plongeant le corps détectable (7) dans de l'acide.

22. Procédé selon la revendication 20, **caractérisé en ce que** la rugosité de ladite surface extérieure vaut environ 5 - 50 µm.
